# EUROPEAN PATENT SPECIFICATION

(11) **EP 3 061 480 B1**
(45) Date of publication and mention of the grant of the patent: **08.07.2020**
(21) Application number: 15382085.7
(22) Date of filing: 27.02.2015
(51) Int. Cl.: A61M 13/00

(54) **Gas insufflator**
Medizinische Gaseinblasvorrichtung
Insufflateur à usage médical

(43) Date of publication of application: 31.08.2016
(73) Proprietor: Costovici, Nicolas Anthony, 17480 Roses (ES)
(72) Inventor: Costovici, Nicolas Anthony, 17480 Roses (ES)
(74) Representative: Gallego Jiménez, José Fernando

(56) References cited:
- US-A- 5 476 447
- US-A1- 2010 106 080
- US-A1- 2012 130 304
- US-A1- 2012 157 770
- US-A1- 2014 350 384

## Description

### Subject of the invention.

The subject of the present invention is a gas insufflator applicable in the distension of the colon of a patient, comprising an electronic control device for adjusting and selecting of various selectable configurations by a radiologist and automatically controlling the gas pressure to be applied during a procedure and also automatically managing the possible excess gas pressures that can be produced during the distension of the colon.

### Field of application of the invention.

This insufflator is applicable in the distension of the colon of a patient during the performance of clinical tests, for example, CT colonoscopy or virtual colonoscopy.

### Prior art.

CT colonoscopy or virtual colonoscopy is performed very often with the help of an automatic insufflator generally using CO2 to insufflate the colon and achieve an appropriate distension during the CT acquisition.

The existing automated insufflators maintain a constant pressure inside the colon during their use and they keep the colon open and distended. These insufflators have a control device with two principal parameters, being in order of priority: the pressure produced inside the colon and the maximum allowable flow rate of gas. The flow rate will be influenced by the pressure and it is a secondary parameter.

In order to perform a CT colonoscopy, the patient receives a colon preparation to have a clean colon. The preparation usually makes use of certain laxatives. Since the preparation is not perfect, the patient also generally absorbs certain contract agents to label the residues remaining in the colon. In this way, the radiologist can distinguish between stool and a polyp or other pathology.

The preparations are very different among countries and even within the same country or region, because radiologists are using different preparations.

The diagnostic software extracts the colon in a 3D model allowing the radiologist to see the colon and make a diagnosis in 3D (virtual colonoscopy software), and there are even some cleaning algorithms. In other words, the diagnostic software is able to clean the colon (if the stools and liquids have been labeled with contrast agent) and present an empty colon to the radiologist, even if the two initial points had liquids or stools.

This new colon diagnostic software functionality allows a new, "light" preparation of the colon, using lesser doses of laxative or even a "fecal labeled" preparation.

The consequence is that the insufflators have to insufflate dirty colons, having much more liquid and stool than a conventional preparation with high doses of laxative.

When the colon has a "low" preparation, the insufflation is disturbed by the liquids and the stools. A normal insufflator will have great difficulty in performing the insufflation. It is necessary to use higher pressures, increasing the pressure established in the insufflator, or the insufflation will last a long time and probably will not be good enough.

If the insufflation is not good or if there are collapsed segments, the radiologist have various difficulties in the diagnosis. The automatic extraction of the colon image which is done by the diagnostic software will be incomplete. The CT colonoscopy diagnostic software extracts only the zones insufflated with CO2 or air and the fact that the colon is not totally distended with collapsed zones will mean that the software only extracts some segments and the radiologist will have to manually connect different segments and finally get an incomplete 3D colon. This segmented 3D of the colon will be incomplete with all the parts between the segments that are not visible in 3D to the radiologist. He will have to read in 2D the "closed" segments, which will cause further loss of time. Incidentally, for the finding of polyps, a large number of clinical trials have shown that the 3D reading has better performance and precision. Thus, the precision of the natural form of diagnosis will be affected by a poor distension of the colon.

If the pressure is not elevated, the flow of the machine will be halted in each segment of the colon that is full of liquid or stool and the colon will probably have a poor distension and collapsed segments at the time of acquisition of the CT.

When the insufflator is set at "normal" pressure and a poor status of the insufflation process is detected, the solution is to increase the insufflation pressure to "high" or to start directly with the "high" pressure and maintain the high pressure during the entire insufflation procedure.

The consequence is that the colon has to experience a high pressure continually, which causes various inconveniences:
- Risk of spasm is greater because the colon reacts in natural manner to persistent high pressure by spasm. The spasm will probably close a portion of the colon and create collapsed segments with the mentioned consequences.
- The patient is awake (without anesthesia) so that the constant higher pressure will cause discomfort or even pain. The discomfort and pain are in proportion to the pressure level.
- In the case of a pathology, the high pressure increases the risk of perforation and accidents (diverticulosis, stenosis, tumors, etc.).

The applicant is unaware of the existence of any prior art able to solve the stated problems in satisfactory manner.

US 2014/350384 A1, US 2012/157770 A1, US 5 476 447 A, US 2010/106080 A1 and US 2012/130304 A1 describe different systems including medical insufflation devices for patients.

### Description of the invention

The gas insufflator applicable in the distension of the colon of a patient comprises a control device allowing the selection of different operating configurations, using a "normal" or constant nominal pressure in each of them, and optionally during a predetermined time, only when necessary, certain electronically controlled and safe "high" pressures.

The gas insufflator of the invention, applicable in distension of the colon, is of the type described in the preamble of claim 1, comprising a control device for at least one valve supplying gas at pressure to a conduit insertable in the colon of a patient and at least one valve for releasing pressure in said insertable conduit; and a meter of the gas pressure in the insertable conduit.

According to the invention, the control device comprises:
a) - a microprocessor which is programmed with a series of operating configurations for application of gas during the process of distensioning of the colon of a patient; wherein each operating configuration includes:
   - parameters relative to a "normal" pressure or nominal pressure of the gas being supplied via the insertable conduit in said configuration,
   - at least one allowable value of excess pressure of the gas in the insertable conduit in said configuration and
   - predetermined periods of time for the automatic releasing of each allowable value of excess pressure in said configuration;
b) - a user interface with several push buttons for entering the parameters of each operating configuration and gas application during the process of distensioning of the colon and selection and activation of the configuration to be used in the process of distensioning of the colon of each patient.

The user interface comprises a manual actuation key for the punctual supply of an excess gas pressure of a predetermined value during a controlled period of time and automatic releasing of the excess pressure after said controlled period of time.

This control device allows the insufflator to use a specific operating configuration which changes the behavior of the insufflator and/or use a specific key which provides a controlled and temporary excess pressure on demand.

In each operating configuration, the insufflator will change according to the cleanness of the colon and the zones or resistance or excess pressure detected. The microprocessor acts on certain valves regulating the supply and release of pressure depending on the configuration chosen by the radiologist via the interface, which has different operating configurations of the control device of the insufflator depending on the degree of cleanness of the colon and the radiologist can adjust the "cleanness" according to the preparation and degree of cleanness of the patient's colon.

The control device acts on the software which regulates the machine. When the colon is very clean, the machine will insufflate gas to reach the set pressure and stop upon reaching the established pressure. The machine will not persist, which would produce a comfortable insufflation for the patient.

In case of resistance or excess pressure, the machine will immediately release the excess pressure from the patient to maintain a constant pressure without any kind of excess pressure.

If the radiologist has configured the insufflator for an operating configuration corresponding to a "dirty colon", the insufflator will have a more persistent behavior and will be more aggressive, so that the gas insufflated can cross the obstructed zones of the colon. This configuration will allow excess pressure for short periods of time to push the stool or get through a segment full of liquid.

This control device will automatically allow certain short and controlled excess pressures instead of the prior art, which is to increase the pressure during the entire radiological examination of the colon. Thus, the pressure will increase only for some moments during the procedure, when the insufflator is encountering a resistance to the insufflation or in another place when necessary.

The microprocessor uses an algorithm to manage short and limited excess pressures, so as to avoid any perforation of the colon, any pain for the patient, and any accidents. This functionality is impossible to reproduce manually. It has to be programmed in the regulating device of the insufflator, and the insufflator has to detect the point of resistance and adapt its behavior. Thus, the algorithm has to respond prior to the pressure increase due to a dirty zone of the colon blocking the insufflation flow.

The radiologist can manually create a short excess pressure by the use of a specific key of the interface, known as the "unblock" key, which increases the pressure for a moment and then releases the excess pressure.

The radiologist can start the insufflation with an intermediate operating configuration of the control device (between "very clean" and "dirty") and during the insufflation he can change the configuration to "dirty" if he realizes that the colon is too long to insufflate and the distension is not progressing as it should. He can also make an "exploratory trip" to verify that the cause of the lack of progress of the insufflation is due to the preparation and not to a pathology.

The radiologist can also use the "unblock" key at any time to act at one point with an excess pressure and cross a dirty zone of the colon.

Accordingly, the control device has various operating configurations depending on the degree of cleanness of the colon between "clean colon" and "dirty colon" and a manual "unblock" key to actuate electronically and safely the excess pressure to be applied.
- Operating configuration established in "clean colon":
   Insufflator set to "Normal" pressure => Detection of a resistance or excess pressure => automatic and immediate releasing of excess pressure.

The control device will control the valve or valves for releasing the excess pressure of the insufflator and not allow any excess pressure during the entire procedure.
- Operating configuration established in "dirty colon ":
   Insufflator set to "Normal" pressure => Detection of a resistance or excess pressure => waits for a period T leaving the excess pressure => After the period T, automatic and immediate releasing of excess pressure to arrive at the "Normal" pressure setting.

The operating configuration of the control device can be set between "clean colon" and "dirty colon" and the setting will affect the period of time T of immediate release for a longer established period.
- If the position set is "dirty colon" and the insufflation continues to be blocked or if the operator decides to cross a dirty zone:
   Insufflation established in "unblock" key => operator presses "normal pressure".

The "unblock" key causes the pressure to increase for a controlled period and then releases the excess pressure to return to normal pressure. The control device will automatically control the excess pressure and prevents the operator from using the "unblock" key again until the two points recover the "normal pressure" and after a rest period.

### Description of the figures.

To supplement the specification being made and in order to facilitate the understanding of the characteristics of the invention, the present specification is accompanied by a set of drawings which represent the following, in illustrative but not limiting manner:
- Figure 1 shows a schematic view of one sample embodiment of the gas insufflator applicable in the distensioning of the colon of a patient.
- Figures 2 and 3 each show schematic views of two screens of the user interface of the control device.

### Preferred embodiment of the invention.

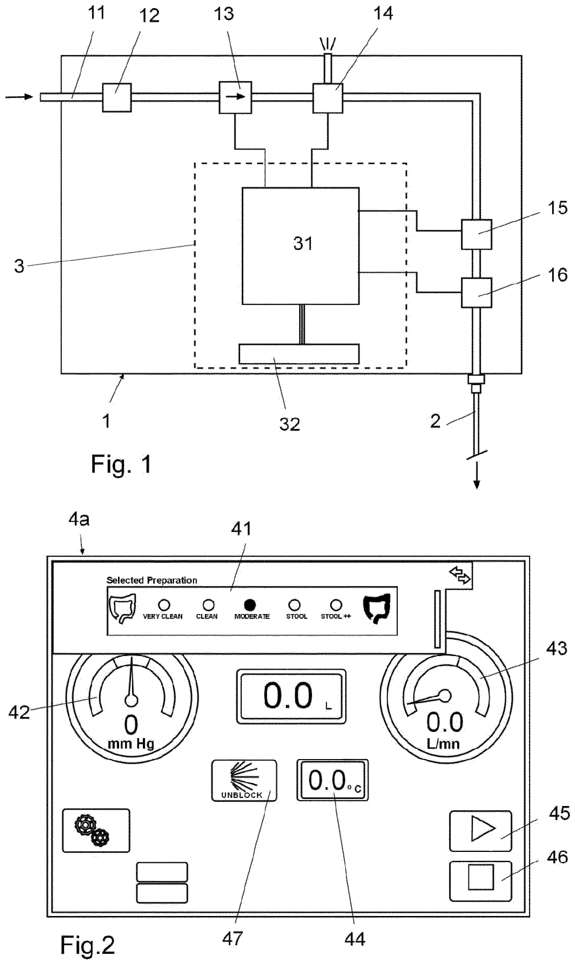

In the sample embodiment shown in Fig. 1, the insufflator (1) comprises a supply conduit (11) for pressurized gas to an insertable conduit (2) in the colon of a patient for the delivery of the gas and the distensioning of the colon.

In said supply conduit (11) there are installed: a regulator (12) of the entry pressure of a gas being supplied; a valve for supply (13) of gas to the insertable conduit (2); a pressure release valve (14); a flow rate meter (15) and a pressure meter (16).

The insufflator (1) comprises a control device (3) which includes: - an microprocessor (31) and a user interface (32).

The microprocessor (31) has the duty of controlling the gas supply valve (13) and gas pressure release valve (14), said microprocessor receiving from the flow rate meter (15) and the pressure meter (16) the flow rate readings of the gas which is being supplied to the patient, and the pressure present inside the insertable conduit and therefore inside the patient's colon.

The microprocessor is programmed with a series of configurations for application of gas during the process of distensioning of the patient's colon. In Figure 2 one can see a screen (4a) of the user interface (32) during the selection of one of the configurations shown in a window (41) and programmed for different degrees of cleanness of the patient's colon: "very clean", "clean", "moderate", "stool", "stool ++".

In this screen (4a) one can also see certain indicators (42, 43) of pressure and flow rate, an indicator (44) of the temperature of the gas which can be used in the event that the insufflator has means of heating the gas supplied, start/stop buttons (45, 46), and an "unblock" key (47).

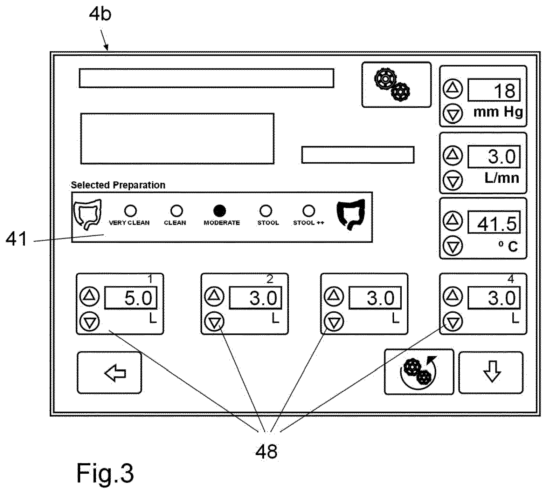

In Figure 3 is shown a screen (4b) of the user interface with a series of push buttons (48) for setting the different parameters: "normal" pressure or nominal pressure of the gas being supplied; allowable values of excess pressure, predetermined periods of time for the automatic release of each allowable value of excess pressure, and temperature of the gas to be used in each of the configurations selectable by the radiologist and shown in the window (41).

Once the settings have been made for each configuration, the control device (3) acts on the insufflator and controls the valves to work at a "normal" pressure for the selected configuration and automatically eliminate any possible excess pressure which may occur after an also predetermined period of time.

## Claims

1. Gas insufflator applicable in the distension of the colon of a patient, comprising: an electronic control device (3) for at least one valve (13) supplying gas at pressure through a supply conduit (11) to a conduit (2) insertable in the colon of a patient and at least one valve (14) for releasing pressure in said supply conduit (11); and a meter (16) of the gas pressure in the insertable supply conduit (11); **characterized in that** said control device (3) comprises:
a) - a microprocessor (31) which is programmed with a series of operating configurations, each of said operating configurations corresponding to a respective different degree of cleanness of the patient's colon, for application of gas during the process of distensioning of the colon of a patient; wherein each operating configuration includes:
- parameters relative to a "normal" pressure or nominal pressure of the gas being supplied via the insertable conduit (2),
- at least one allowable value of excess pressure of the gas in the insertable conduit (2) and
- predetermined periods of time for the automatic releasing of each allowable value of excess pressure in said configuration;
b) - a user interface (32) for entering the parameters of each operating configuration of the insufflator during the process of distensioning of the colon; and for the selection and activation of the configuration to be used in the process of distensioning of the colon of each patient.

2. Insufflator according to claim 1, **characterized in that** the user interface comprises an "unblock" key (47) for manual actuation for the punctual supplying of an excess pressure of gas of a predetermined value during a controlled period of time and the automatic releasing of the excess pressure after said controlled period of time.

3. Insufflator according to any one of the preceding claims, **characterized in that** the user interface (32) has a screen (4a) for selection of one of the operating configurations shown in a window (41) and programmed for different degrees of cleanness of the patient's colon.

4. Insufflator according to claim 3, **characterized in that** the screen (4a) also comprises indicators (42, 43) of pressure and flow rate of the gas in the supply conduit.

5. Insufflator according to any one of claims 1 and 2, **characterized in that** the user interface (32) has a screen (4b) with a series of push buttons (48) for setting the different parameters: "normal" pressure or nominal pressure of the gas being supplied; allowable values of excess pressure and predetermined periods of time for the automatic releasing of each allowable value of excess pressure, in each of the selectable operating configurations.

## Patentansprüche

1. Gasinsufflator, anwendbar bei der Ausdehnung des Dickdarms eines Patienten, umfassend: eine elektronische Steuervorrichtung (3) für mindestens ein Ventil (13), das Gas mit Druck durch eine Versorgungsleitung (11) zu einer Leitung (2) zuführt, die in den Dickdarm eines Patienten eingeführt werden kann, und mindestens ein Ventil (14) zum Ablassen des Drucks in die Versorgungsleitung (11); und ein Messgerät (16) des Gasdrucks in der einführbaren Versorgungsleitung (11); **dadurch gekennzeichnet, dass** die Steuervorrichtung (3) umfasst:
a) - einen Mikroprozessor (31), der mit einer Reihe von Betriebskonfigurationen programmiert ist, wobei jede der Betriebskonfigurationen einem jeweils unterschiedlichen Reinheitsgrad des Dickdarms des Patienten entspricht, zum Anwenden von Gas während des Prozesses der Ausdehnung des Dickdarms eines Patienten; wobei jede Betriebskonfiguration einschließt:
- Parameter in Bezug auf einen "normalen" Druck oder Nenndruck des Gases, das über die einführbare Leitung (2) zugeführt wird,
- mindestens einen zulässigen Wert von überschüssigem Druck des Gases in die einführbare Leitung (2) und
- vorbestimmte Zeiträume für die automatische Freigabe jedes zulässigen Wertes des überschüssigen Drucks in der Konfiguration;
b) - eine Benutzerschnittstelle (32) zum Eingeben von Parametern jeder Betriebskonfiguration des Insufflators während des Prozesses der Ausdehnung des Dickdarms; und für die Auswahl und Aktivierung der zu verwendenden Konfiguration in dem Prozess der Ausdehnung des Dickdarms jedes Patienten.

2. Insufflator nach Anspruch 1, **dadurch gekennzeichnet, dass** die Benutzerschnittstelle einen "Entblockungs"-Taster (47) zur manuellen Betätigung für die punktuelle Zufuhr eines überschüssigen Gasdrucks eines vorbestimmten Wertes während eines gesteuerten Zeitraums und die automatische Freigabe des überschüssigen Drucks nach dem gesteuerten Zeitraum umfasst.

3. Insufflator nach einem der vorhergehenden Ansprüche, **dadurch gekennzeichnet, dass** die Benutzerschnittstelle (32) einen Schirm (4a) für die Auswahl einer der Betriebskonfigurationen aufweist, die in einem *Fenster* (41) gezeigt werden und für unterschiedliche Grade der Sauberkeit des Patientendickdarms programmiert sind.

4. Insufflator nach Anspruch 3, **dadurch gekennzeichnet, dass** der Schirm (4a) auch Anzeigen (42, 43) des Drucks und des Durchflusses von Gas in der Versorgungsleitung umfasst.

5. Insufflator nach einem der Ansprüche 1 und 2, **dadurch gekennzeichnet, dass** die Benutzerschnittstelle (32) einen Schirm (4b) mit einer Reihe von Drucktasten (48) zum Einstellen der unterschiedlichen Parameter aufweist: "Normal"-Druck oder Nenndruck des zugeführten Gases; zulässige Werte von übermäßigem Druck und vorbestimmten Zeiträumen zur automatischen Freigabe jedes zulässigen Wertes von übermäßigem Druck in jedem der auswählbaren Betriebsparameter.

## Revendications

1. Insufflateur de gaz applicable dans la distension du côlon d'un patient, comprenant : un dispositif de contrôle électronique (3) pour au moins une vanne (13) fournissant du gaz sous pression à travers un conduit d'alimentation (11) à un conduit (2) insérable dans le côlon d'un patient et au moins une vanne (14) pour la libération de la pression dans ledit conduit d'alimentation (11) ; et un compteur (16) de la pression de gaz dans le conduit d'alimentation (11) insérable ; **caractérisé en ce que** ledit dispositif de contrôle (3) comprend :
a) - un microprocesseur (31) qui est programmé avec une série de configurations d'exploitation, chacune desdites configurations de d'exploitation correspondant à un différent degré de propreté respectif du côlon du patient, pour l'application de gaz pendant le processus de distension du côlon d'un patient ; dans lequel chaque configuration d'exploitation comporte :
- des paramètres relatifs à une pression « normale » ou à une pression nominale du gaz étant fourni via le conduit insérable (2),
- au moins une valeur admissible d'excès de pression du gaz dans le conduit (2) insérable et
- des périodes de temps prédéterminées pour la libération automatique de chaque valeur admissible d'excès de pression dans ladite configuration ;
b) - une interface utilisateur (32) pour la saisie des paramètres de chaque configuration d'exploitation de l'insufflateur pendant le processus de distension du côlon ; et pour la sélection et l'activation de la configuration à utiliser dans le processus de distension du côlon de chaque patient.

2. Insufflateur selon la revendication 1, **caractérisé en ce que** l'interface utilisateur comprend une touche de « déblocage » (47) pour un actionnement manuel pour la fourniture ponctuelle d'un excès de pression de gaz d'une valeur prédéterminée pendant une période de temps contrôlée et la libération automatique de l'excès de pression après ladite période de temps contrôlée.

3. Procédé selon l'une quelconque des revendications précédentes, **caractérisé en ce que** l'interface utilisateur (32) a un écran (4a) pour la sélection de l'une des configurations d'exploitation montrées sur une *fenêtre* (41) et programmées pour différents degrés de propreté du côlon du patient.

4. Insufflateur selon la revendication 3, **caractérisé en ce que** l'écran (4a) comprend également des indicateurs (42, 43) de pression et de débit du gaz dans le conduit d'alimentation.

5. Insufflateur selon l'une quelconque des revendications 1 et 2, **caractérisé en ce que** l'interface utilisateur (32) a un écran (4b) avec une série de boutons poussoirs (48) pour le réglage des différents paramètres : pression « normale » ou pression nominale du gaz étant fourni ; valeurs admissibles d'excès de pression et périodes de temps prédéterminées pour la libération automatique de chaque valeur admissible d'excès de pression, dans chacune des configurations d'exploitation sélectionnables.
